# EUROPÄISCHE PATENTANMELDUNG

(11) **EP 1 031 378 A2**
(43) Veröffentlichungstag der Anmeldung: **30.08.2000**
(21) Anmeldenummer: 00250063.5
(22) Anmeldetag: 25.02.2000
(51) Int. Cl.: B01J 23/75, B01J 37/34, C07C 1/04

(54) **Kobaltkatalysator für die Fischer-Tropsch-Synthese**

(30) Priorität: 26.02.1999 DE 19910151
(71) Anmelder: Institut für Angewandte Chemie Berlin-Adlershof E.V., 12489 Berlin (DE)
(72) Erfinder: Baerns, Prof. Dr.Manfred, 14195 Berlin (DE)
(74) Vertreter: Walter, Wolf-Jürgen

(57) **Zusammenfassung**

Die Erfindung betrifft einen Cobaltkatlysator für die Fischer-Tropsch-Synthese, der über ein plasmagestütztes Verfahren hergestellt wird. Eine feste organische Cobaltverbindung, ausgewählt aus der Gruppe, bestehend aus Cobaltsalzen organischer Säuren, Cobaltalkoholat, Cobalt-Kronenverbindungen, Cobaltacetylacetonat, Cobaltchelat, Cobaltcarbonyle, Cobaltcarbonylkomplexe und Gemischen davon, wird in Gegenwart eines pulverförmigen, calcinierten Trägermaterials bei einer Temperatur von 15 bis 150 °C einem Mikrowellenplasma von 50 bis 1200 W bei einer Frequenz von 2,46 GHz für einen Zeitraum von 30 Minuten bis 50 Stunden bei einem Druck von 0,1 bis 5 mbar unter gleichzeitiger Bewegung des Trägermaterials ausgesetzt. Der Katalysator hat eine sehr gute Selektivität.

## Beschreibung

Die Erfindung betrifft einen verbesserten Kobaltkatalysator, der bei der Fischer-Tropsch-Synthese zur Herstellung langkettiger Kohlenwasserstoffe aus CO und H₂ eingesetzt werden kann.

Es sind eine Vielzahl von Verfahren bekannt, um auf Substraten durch Plasmaverfahren (z.B. Sputtern) metallische Schichten bzw. Metalloxidschichten aufzubringen.

Aus Thin Solid Films 305 (1997) 210-218 ist die Abscheidung von Cobalt und Co-Fe-Cr auf Glasoberflächen durch Ultraschallanregung von Cobaltacetylacetonat bekannt, insbesondere zur Herstellung von Schichten mit besonderen optischen Eigenschaften.

Die plasmagestützte metallorganische Dampfphasenabscheidung von Cobaltacetylacetonat wurde zur Herstellung von oxidischen Cobaltschichten auf planaren Substraten genutzt [Jpn.J.Appl.Phys., Vol. 32 (1993) S. L1448-L1450].

Weiterhin ist die Herstellung metallischer Cobaltschichten durch Hochdrucksputtern bei 1 Torr und einer Substrattemperatur von 720 K aus J.Vac.Sci.Technol. A16, 759 (1998) bekannt.

Der Erfindung liegt die Aufgabe zugrunde, neu strukturierte Kobaltverbindungen auf Trägern als Katalysator bereitzustellen.

Erfindungsgemäß ist der neue Katalysator dadurch gekennzeichnet, daß er hergestellt wird, indem eine feste organische Cobaltverbindung, ausgewählt aus der Gruppe, bestehend aus Cobaltsalzen organischer Säuren, Cobaltalkoholat, Cobalt-Kronenverbindungen, Cobaltacetylacetonat, Cobaltchelat, Cobaltcarbonyle, Cobaltcarbonylkomplexe und Gemischen davon, in Gegenwart eines pulverförmigen, calcinierten Trägermaterials bei einer Temperatur von 15 bis 150 °C einem Mikrowellenplasma von 50 bis 1200 W bei einer Frequenz von 2,46 GHz für einen Zeitraum von 30 Minuten bis 50 Stunden bei einem Druck von 0,1 bis 5 mbar unter gleichzeitiger Bewegung des Trägermaterials ausgesetzt wird.

Im Zusammenhang mit der Erfindung sind "diskrete Metallteilchen" solche Teilchen, die im wesentlichen keine zusammenhängende Schicht ausbilden sondern einzeln und/oder inselförmig in gewissen Abständen voneinander auf dem Trägermaterial vorliegen. Die Abstände können vorzugsweise 4-6 nm betragen; sie können auch bis zu 50 nm betragen.

Unter dem Begriff "Metallteilchen" werden Teilchen reiner Metalle sowie Metalloxidteilchen verstanden.

Das Trägermaterial ist ein pulver- oder granulatförmiger Körper mit unregelmäßiger Oberfläche, vorzugsweise ein feinteiliges pulverförmiges Material. Die Korngrößen können zwischen wenigen Mikrometern bis hin zu mehreren Millimetern liegen, z.B. 5-10 µm bis 5 mm. Es kann auch ein Material mit regelmäßiger Oberfläche sein, wie z.B. Plättchen.

Das vorzugsweise pulverförmige Material wird mindestens während eines Zeitabschnittes der Plasmaeinwirkung bewegt, vorzugsweise während des gesamten Abscheidungsprozesses, um eine gleichmäßige Verteilung der Metallpartikel auf der Substratoberfläche zu gewährleisten.

Als Trägermaterial kann ein anorganisches Oxid wie beispielsweise TiO₂, ein Zeolith, ein Silicat, ein Alumosilicat, eine Aktivkohle, Kaolin, Tonerde, Talk, Kieselgel, Kieselgur oder ein Gemisch davon eingesetzt werden, wenn beispielsweise ein katalytisch aktives Material hergestellt werden soll. Es können auch andere Substrate für diese und andere Verwendungszwecke verwendet werden, wie z.B.
Gläser oder Substrate aus der Halbleiterindustrie (Silicium, GaAs, Al₂O₃).

Als organische Cobaltverbindung wird eine bei 50 bis 200 °C verdampfbare organische Verbindung mit daran gebundenen oder angelagerten Cobaltmetall eingesetzt. Das bedeutet, daß der Begriff "organische Cobaltverbindung" nicht im strengen Sinne einer Kohlenstoff-Metall-Bindung verwendet wird, sondern es können außer Salzen organischer Säuren, Alkoholaten, Kronenverbindungen usw. auch Cobaltacetylacetonate, Cobaltchelate, Cobalt-Carbonylkomplexe verwendet werden. Bevorzugt sind Cobaltacetylacetonate, -chelate und -alkoholate.

Ein besonderes Merkmal der Erfindung besteht darin, daß die organische Cobaltverbindung in fester Form im Verfahren eingesetzt wird und nicht als Flüssigkeit oder Dampf in das Reaktorgefäß eingeleitet werden muß. Die für die Überführung der organischen Cobaltverbindung in die Gasphase erforderliche Temperatur von 50 bis 200 °C wird vorteilhaft durch die Mikrowellenstrahlung erzeugt.

Die organische Cobaltverbindung wird vorteilhaft in einer tablettierten Form zusammen mit dem pulverförmigen Trägermaterial dem Mikrowellenplasma ausgesetzt. Die organische Cobaltverbindung kann auch in Pulverform eingesetzt werden.

Erfindungsgemäß wird ein Niederdruck-Mikrowellenplasma mit einer Leistung von 50 bis 1200 W eingesetzt, vorzugsweise 100 bis 500 W. Das Mikrowellenplasma hat vorzugsweise eine Frequenz von 2,46 GHz. Der für die Plasmaerzeugung erforderliche Druck in einer Vakuumkammer liegt vorzugsweise bei 0,01 bis 100 mbar und kann gegebenenfalls nach dem Zünden des Plasmas bis auf 1 bar erhöht werden.

Das Plasma kann ein Sauerstoffplasma, ein Wasserdampfplasma oder ein Gemisch beider sein, wobei diesen Plasmen inerte Trägergase, wie z.B. Argon oder Helium zugesetzt werden können. Es kann auch allein mit inerten Plasmen gearbeitet werden.

Die Größe der Metallteilchen auf der Oberfläche des Trägermaterials kann durch Bewegung des Trägermaterials, die jeweiligen Partialdrücke, die Zeit der Plasmabehandlung, die Plasmaleistung, die Menge der metallorganischen Verbindung so gesteuert werden, daß Teilchen mit Durchmessern unterhalb 10 nm, vorzugsweise 4-6 nm auf die Oberfläche aufgetragen werden.

Vorzugsweise wird die Plasmabehandlung für einen Zeitraum aufrechterhalten, in dem auf wenigstens 50 % der Oberfläche des Trägermaterials keine zusammenhängende Fläche aus Metallpartikeln gebildet wird, sondern im wesentlichen nur diskrete Metallteilchen mit Durchmessern von ca. 4-10 nm abgeschieden werden.

Es wurde gefunden, daß der erfindungsgemäße Katalysator gegenüber einem auf herkömmliche Weise durch Imprägnierung hergestellten Cobaltkatalysator nach seiner Reduktion unter gleichen Bedingungen bei der Herstellung längerkettiger Kohlenwasserstoffe nach der Fischer-Tropsch-Synthese sowohl einen um wenigstens 20 % erhöhten Umsatz zeigt als auch deutlich verbesserte Selektivitäten für Kohlenwasserstoffe mit 7 und mehr Kohlenstoffatomen in der Kette.

Die Herstellung des Katalysators kann in einer Vorrichtung erfolgen, die sich in einer Vakuumkammer befindet und aus einem Rotor 1 mit horizontal daran angeordneter Welle 2 mit einer Dichtung 3 zum Rotor 1 und einem um die Welle 2 angeordneten und mit ihr fest verbundenen Reaktionsbehälter 4 besteht, wobei die Wandungen des Reaktorbehälters 4 einen ausreichenden Abstand zur Welle 2 aufweisen, um ohne Kontakt mit der Welle 2 bei Rotation des Reaktorbehälters 4 ein stückiges oder feinteiliges Substrat 5 aufnehmen zu können, und um ein um die Welle herum angeordnetes Drahtgeflecht 6 zur Erhöhung der Plasmadichte auf diesen Bereich aufnehmen zu können.

Der Rotor 1 ist über eine Drehdurchführung, die sich in der Wand der Vakuumkammer befindet, mit einem außerhalb der Kammer befindlichen Motors gekoppelt.

Zwischen der Welle 2 und der Dichtung 3 befindet sich ein schmaler Spalt über den das Volumen im Reaktorbehälter 4, welches ein Teil des Gesamtvolumens der Vakuumkammer ist, abgepumpt werden kann. Eine Öffnung 8 in dem Reaktorbehälter 4, die im wesentlich zur Befüllung dient, kann zur Vermeidung des Austritts von Trägermaterial bzw. metallorganischer Verbindung in festem Zustand mit einer Metallfolie oder mit einer anzuflanschenden Fritte abgedeckt werden. Die Welle 2 kann auch Bohrungen aufweisen, durch die das Trägergas dem Reaktorbehälter 4 zugeführt werden kann, oder das Trägergas wird über separate Leitungen in die Vakuumkammer eingeführt.

Durch das Drahtgeflecht 6, das vorteilhaft aus Edelstahl besteht, der beispielsweise spiralförmig oder schleifenartig als durchgehender Draht geformt ist, wird wahrscheinlich ein hohlkathodischer Plasmaeffekt bewirkt, so daß durch entsprechende Steuerung ein Mikrowellenplasma nur im Inneren des Reaktorbehälters 4 brennen kann. Das Drahtgeflecht kann auch aus anderem Metall bestehen, z.B. Cu, Ni usw..

Besondere Vorteile der Herstellung des Katalysators auf die genannte Weise sind:
(1) Eine für ein Vakuumbeschichtungsverfahren einfache Handhabbarkeit
(2) Unterdrückung der Kontamination der Vakuumkammer, da bei herkömmlichen Plasmaverfahren, bei denen metallorganische Verbindungen verwendet werden, diese zunächst mit einem Lösungsmittel in die flüssige Phase überführt werden müssen und anschließend beispielsweise mit einem teuren beheizbaren Masseflußregler gasförmig in die Vakuumkammer eingeleitet werden.
(3) Effektives Beschichten, da durch die unmittelbare Nähe der organischen Cobaltverbindung zu dem zu beschichtenden Träger, kürzere Prozeßzeiten erforderlich sind. Es wird eine hohe Konzentration der gasförmigen metallorganischen Komponente in der Nähe des Trägermaterials erreicht und die eingesetzte Menge metallorganischer Substanz effektiv für die Beschichtung ausgenutzt. Bei herkömmlichen Verfahren entsprechend (2) wird ein Teil der in der Vakuumkammer befindlichen gasförmigen metallorganischen Verbindung unzersetzt über die Vakuumpumpe abgesaugt bzw. innerhalb der Kammer an Stellen zersetzt, wo sich kein zu beschichtenes Trägermaterial bzw. Substrat befindet.
(4) Universelle Einsatzmöglichkeit, da das Trägermaterial und die organische Cobaltverbindung leicht zu substituieren sind.
(5) Skalierbarkeit bezüglich größerer Einwaagen, was für einen industriellen Einsatz bedeutsam ist.

Die Erfindung soll nachstehend durch Beispiele näher beschrieben werden.

Die Vorrichtung zur plasmagestützten Abscheidung von Metall/Metalloxidteilchen besteht aus einer Vakuumkammer, die auf 10⁻⁵ mbar abgepumpt werden kann, und in der ein horizontal drehbarer Reaktorbehälter bzw. Glaszylinder auf einer Welle angebracht ist. Die Welle steht über eine Öffnung des Glaszlinders mit einem Rotor in Verbindung. Auf die Welle ist ein Drahtgeflecht aufgeschoben.

Auf den Wandungen im Inneren des horizontal angeordneten Glaszylinders liegt das Substrat, das entweder stückig oder ein teilchenförmiges Pulver sein kann. Weiterhin wird in den Glaszylinder die feste metallorganische Verbindung beispielsweise in Form eines Preßkörpers eingebracht.

Nach dem Abpumpen der Vakuumkammer wird im Falle eines Sauerstoffplasmas Sauerstoff so lange zudosiert, bis sich bei laufender Vakuumpumpe ein Druck von etwa 1 mbar einstellt. Bei gleichzeitiger horizontaler Rotation des Reaktorbehälters, bei dem die seitliche Beladungsöffnung mit einer Metallfolie oder mit einer angeflanschten Fritte abgedeckt werden kann, wird ein mikrowellenangeregtes Sauerstoffplasma erzeugt.

Die Rotation des Glaszylinders erfolgt mit ca. 10 bis 100 Umdrehungen pro Minute.

Die organische Cobaltverbindung wird durch die Mikrowelleneinstrahlung und die Plasmawirkung erwärmt und in die Gasphase überführt. Hierbei zersetzt sich die organische Cobaltverbindung durch die Einwirkung des sauerstoffhaltigen Plasmas, wobei der metallische Teil als Oxid auf dem Trägermaterial abgeschieden und der organische Teil der Verbindung größtenteils von der Vakuumpumpe abgesaugt wird.

Es wird ein kontinuierliches Mikrowellenplasma von 120 W (f = 2,46 GHz) verwendet, wobei die Leistung 120 W beträgt.

### Beispiel 1

In einer Vakuumkammer wurde in der erfindungsgemäßen Vorrichtung bei etwa 1 mbar eine Menge von 7 g calciniertem TiO₂-Pulver mit einer Teilchengröße von 5 nm unter Zusatz von 3 g Cobalt(III)acetylacetonat für einen Zeitraum von 16 Stunden in einem kontinuierlichen mikrowellenangeregten Sauerstoffplasma behandelt. Die Frequenz des Plasmas betrug 2,46 GHz und die Leistung 120 W. Die Rotationsgeschwindigkeit des Glaszylindes in dem Rezipienten lag bei 30 U/min.

Der dabei hergestellte Katalysator wurde zu Pellets gepreßt (10 MPa, Haltezeit 1 min) und diese anschließend zerstoßen und gesiebt. Die anfallende Fraktion von 255-355 µm wurde in einem Röhrenofen reduziert. Hierzu wurde ein H₂-Strom (Fluß: 30 ml/min; Reinheit 5,0) über den in einem Porzellanbehälter befindlichen Katalysator geführt. Der Katalysator wurde zunächst von Raumtemperatur auf 400 °C (Aufheizgeschwindigkeit 6,2 K/min) aufgeheizt und anschließend bei dieser Temperatur für 12 Stunden belassen.

Danach erfolgte der Einsatz des Katalysators bei der Fischer-Tropsch-Synthese (Herstellung langkettiger Kohlenwasserstoffe aus CO und H₂ mit Cₙ> ) aus CO/H₂ unter folgenden Bedingungen:

| | |
|---|---|
| CO/H₂ | 2/1 |
| p_{gesamt} | 20 bar |
| Tᵣₑₐₖ | 237 °C |
| ICP* (Co) | 5,5 Gew-% |
| GHSV | 1500 h⁻¹. |

| | |
|---|---|
| * ICP = Inductive Coupled Plasma | |

Man erhielt bei einem CO-Umsatz von 41,65 % folgende Selektivitäten

| | | |
|---|---|---|
| S | C₁ | 1,35 |
| S | C₂ | 0,39 |
| S | C₃ | 1,5 |
| S | C₄ | 1,8 |
| S | C₅ | 2,1 |
| S | C₆ | 1,5 |
| S | C₅₊ | 94,8 |

Im Vergleich mit einem durch Imprägnierung hergestellten Cobaltkatalysator, bei dem folgende Selektivitäten bei einem CO-Umsatz von 34,04 % erreicht wurden

| | | |
|---|---|---|
| S | C₁ | 4,75 |
| S | C₂ | 1,23 |
| S | C₃ | 4,02 |
| S | C₄ | 3,08 |
| S | C₅ | 3,86 |
| S | C₆ | 4,7 |
| S | C₅₊ | 86,89 |

zeigt sich deutlich die Überlegenheit des erfindungsgemäß hergestellten Katalysators.

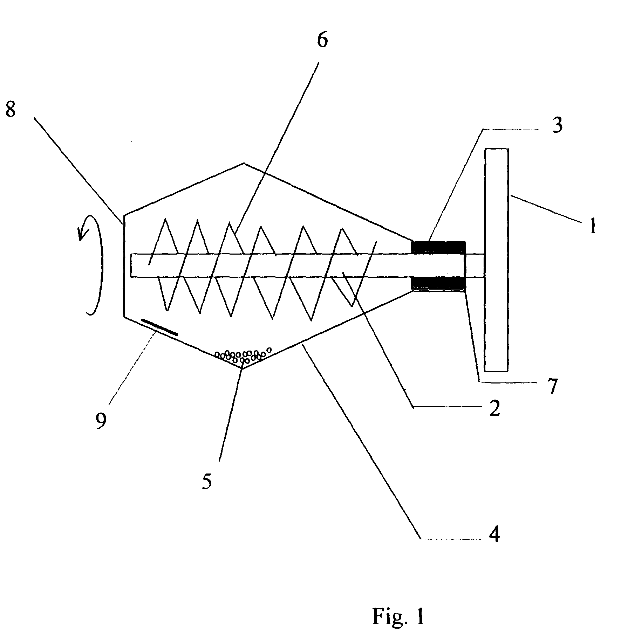

## Patentansprüche

**1.** Cobaltkatalysator für die Fischer-Tropsch-Synthese, hergestellt indem eine feste organische Cobaltverbindung, ausgewählt aus der Gruppe, bestehend aus Cobaltsalzen organischer Säuren, Cobaltalkoholat, Cobalt-Kronenverbindungen, Cobaltacetylacetonat, Cobaltchelat, Cobaltcarbonyle, Cobaltcarbonylkomplexe und Gemischen davon, in Gegenwart eines pulverförmigen, calcinierten Trägermaterials bei einer Temperatur von 15 bis 150 °C einem Mikrowellenplasma von 50 bis 1200 W bei einer Frequenz von 2,46 GHz für einen Zeitraum von 30 Minuten bis 50 Stunden bei einem Druck von 0,1 bis 5 mbar unter gleichzeitiger Bewegung des Trägermaterials ausgesetzt wird.

**2.** Katalysator nach Anspruch 1, dadurch gekennzeichnet, daß die organische Cobaltverbindung Cobaltacetylacetonat ist.

**3.** Katalysator nach Anspruch 1, dadurch gekennzeichnet, daß das Trägermaterial ein anorganisches Oxid, Silicat, Alumosilicat, eine Aktivkohle, Kaolin, Tonerde, Talk, Kieselgel, Kieselgur oder ein Gemisch davon ist.

**4.** Katalysator nach Anspruch 3, dadurch gekennzeichnet, daß das Trägermaterial TiO₂ ist.

**4.** Katalysator nach Anspruch 1, dadurch gekennzeichnet, daß das Mikrowellenplasma ein Niederdruck-Mikrowellenplasma mit einer Leistung von 100 bis 150 W ist.

**5.** Katalysator nach Anspruch 1, dadurch gekennzeichnet, daß die Teilchengröße der Metallteilchen auf der Oberfläche des Trägermaterials in ihrem Durchmesser weniger als 10 nm beträgt.

**6.** Katalysator nach Anspruch 6, dadurch gekennzeichnet, daß die Teilchengröße der Metallteilchen auf der Oberfläche des Trägermaterials bei Durchmessern im Bereich von 4-6 nm liegt.
